(19) Europäisches Patentamt
European Patent Office
Office européen des brevets

(11) Publication number: 0 003 064
A2

# (12) EUROPEAN PATENT APPLICATION

(21) Application number: 78200319.8

(22) Date of filing: 28.11.78

(51) Int. Cl.[2]: A 24 B 15/04
A 24 B 15/027, A 61 K 9/72

(30) Priority: 07.12.77 US 858295

(43) Date of publication of application:
25.07.79 Bulletin 79/15

(84) Designated contracting states:
BE CH DE FR GB LU NL

(71) Applicant: Rabam Limited
P.O. Box 350
Hamilton Bermuda(US)

(72) Inventor: Sherman, Burton, Dr.
184 Elmwood St. Valley Stream
Long Island New York, 11581(US)

(74) Representative: Winkler, Kurt, Dr.
Mellingerstrasse 69
CH-5400 Baden(CH)

## (54) Method for introducing analogs of vitamin A into the respiratory tract of a cigarette smoker.

(57) Natural and synthetic analogs of vitamin A are introduced into the mouth and respiratory tract of a cigarette smoker in the form of droplets (11) or aerosol by incorporating the vitamin A analog in at least one rupturable capsule (13) contained within the mouthpiece end or over the entire length of a cigarette (1), which may also be filtered. The capsules are rupturable under the influence of pressure or heat. The heat can be derived from the hot tobacco smoke or directly from the burning tobacco charge.

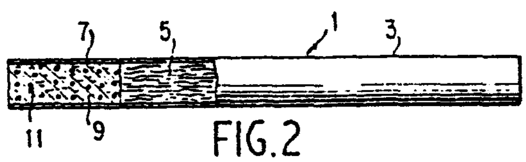

FIG.2

EP 0 003 064 A2

Croydon Printing Company Ltd.

# METHOD FOR INTRODUCING ANALOGS OF VITAMIN A INTO THE RESPIRATORY TRACT OF A CIGARETTE SMOKER

This invention relates to a method for delivering retinoids into the respiratory tract of humans, and especially cigarette smokers, in order to enhance the prophylactic and therapeutic effects of the retinoid substances. More particularly, this invention relates to a method for introducing the congeners derivatives and analogs of vitamin A, both naturally occurring and synthetically produced directly to the respiratory tract mucosa to enhance the prophylactic and therapeutic effects of these retinoid substances.

It has been known from U.S. Patents 3,339,558 and 3,525,582, each issued to Nelson J. Waterbury to incorporate vitamin A which has been encapsulated in rupturable capsules into filtered and non-filtered cigarettes in order to introduce the vitamin A, in the form of droplets or aerosol, to the mouth and respiratory tract of the person smoking such cigarette. This invention is directed to an improvement in the method described in the Waterbury patents.

There has been a great deal of scientific literature published in recent years reporting the effects of retinoids, including both the effects of deficiencies of retinoids and applications of large doses of retinoids with respect to the prophylactic and therapeutic effects on the development of cancers, especially

those cancers by polycyclic aromatic hydrocarbons such as those found in cigarette smoke. The evidence accumulated to date, including the studies of the present inventor, leave little doubt that the retinoid substances exert favorable effects on many types of experimentally produced cancers and in fact, there is published evidence demonstrating that the retinoids exert a direct topical effect on preventing the formation of tumors in respiratory tract mucosa in vitro.

However, when vitamin A is used as the retinoid substance favorable effects are only demonstrated when large oral mega doses are utilized. However, such large dosages of vitamin A needed to produce blood levels of the vitamin which are adequate to demonstrate a prophylactic or therapeutic response are also so high that they generally produce toxic side effects due to hypervitaminosis A. Specific adverse side effects associated with high dosages of vitamin A, whether in the pure form or as the more conventionally used palmitate form include liver, skin and eye damage. Therefore, to avoid the undesirable side effects associated with the large oral dosages of vitamin A needed to produce adequate blood levels of the vitamin to exhibit the intended prophylactic or therapeutic response there have been developed many new synthetic analogs of vitamin A which have also been shown to have good anti-tumor activity and none or only low toxic side effects when given orally. Nevertheless, while many of these newly developed retinoids are much safer for use than vitamin A, it is still desireable to decrease the dosage required of the retinoid substance to achieve the desired effect.

Still further, for the population at large for which the occurrence of lung cancer and related ailments is highest, namely

cigarette smokers, it would be highly desireable to provide a technique for administering retinoid materials to the affected areas and especially the lung tissue, in a convenient, foolproof and psychologically attractive manner.

This has now been accomplished in accordance with the present invention. This is accomplished by a method in which the retinoid substances which are the analogs of vitamin A are delivered directly to the respiratory tract mucosa to thereby exhibit their beneficial effects. The retinoid substance is delivered in the form of tiny droplets or an aerosol into the mouth and respiratory tract of the smoker whereby the maintenance of good health of a cigarette smoker can be enhanced.

More specifically, the present invention provides a method for introducing natural or synthetic analogs of vitamin A into the mouth and respiratory tract of a smoker by locating a controlled amount of a vitamin A analog in the form of droplets or an aerosol of a stabilized solution or a stabilized suspension uniformly distributed in at least the mouth piece end of a cigarette having a tobacco charge or a similar smoking article, igniting the cigarette and applying suction (smoking) the cigarette whereby the smoke passing through the cigarette entrains the vitamin A analog in the form of tiny droplets or as an aerosal into the mouth and respiratory tract of the smoker.

In an alternative mode according to the present method, vitamin A analog is introduced into the mouth and respiratory tract of a smoker by locating at least one heat or pressure rupturable capsule containing a controlled amount of vitamin A analog in at least the mouth piece end of a cigarette having a tobacco charge

or similar smoking article, rupturing the vitamin A analog containing capsule or capsules immediately before or during smoking to thereby release the vitamin A analog in the form of tiny droplets or an aerosol for exposure to the smoke passing through the cigarette, and smoking the cigarette whereby the tiny droplets or aerosol of the vitamin A analog will be carried in the smoke to the mouth and respiratory tract of the smoker.

In the first mentioned embodiment, the stabilized solutions or suspensions of the vitamin A analog are uniformly distributed throughout the filtering medium of a filter cigarette and optionally, also throughout the tobacco charge. In a non-filtered cigarette the droplets or aerosol of the dispersion or solution of the vitamin A analog is preferably uniformly distributed throughout the tobacco charge. In either case, the size of the droplets or aerosol should be sufficiently small so as to be easily entrained by the tobacco smoke during the smoking process.

In the latter embodiment utilizing encapsulated vitamin A analogs, the retinoid material may be in liquid or vapor form, but is preferably in liquid form. The encapsulating material may be rupturable by application of pressure or by application of heat such as derived from the hot tobacco smoke or the heat of the burning tobacco charge. If only a single capsule is used, it will be located in the mouth end of the cigarette and in a filtered cigarette, it will be located in the filter medium.

When the capsules are pressure rupturable, the capsules will

be ruptured by application of pressure thereto immediately before smoking. When a plurality of pressure rupturable capsules are used, then at least the majority of the capsules and preferably, all of the capsules are ruptured by application of pressure thereto immediately before smoking. When using a plurality of the pressure rupturable capsules, the capsules should be distributed throughout the cigarette with preferably at least the majority of the capsules located in contact with and uniformly distributed throughout the filter medium of a filtered cigarette.

When the vitamin A analog is encapsulated in a heat rupturable capsule, it is preferred to use a plurality of the heat rupturable capsules relatively uniformly distributed over the length of the cigarette whereby the vitamin A analog will be dispensed relatively uniformly over the course of smoking the cigarette. It is possible, however, to also include only a single heat rupturable capsule located at the mouth end of the cigarette and in the filter medium in a filtered cigarette. Preferably a multiplicity of microcapsules formed from heat rupturable walls are utilized.

The vitamin A analogs used in the method of the subject invention may be either naturally ocurring or synthetically produced substances. In particular, the vitamin A analog according to the present invention may take any one of the following forms as indicated by the following formulas:

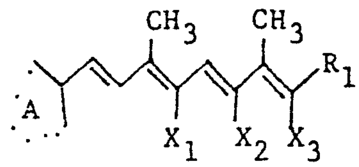

(I)

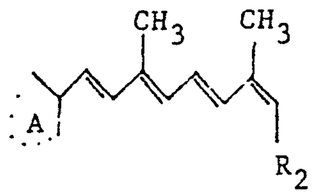

(II)

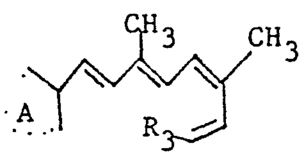

(III)

wherein the ring A, 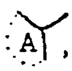, is a five or six membered, mono- or di-unsaturated or aromatic hydrocarbon ring, which may include a nitrogen or oxygen hetero atom in the ring, and which may include one or more substituents selected from the group consisting of lower alkyl lower alkoxy, halogen, lower alkoxyalkyl and lower acyl;

$X_1$, $X_2$ and $X_3$ are each a hydrogen atom or one of them may be a halogen atom; and

R is a member selected from the group consisting of -CHO, -COOR, $-R_2O\overset{O}{\overset{\|}{C}}-R_3$, $-CONHR_4$, $-R_5OR_6$, $-R_7NHCOR_8$, -CHNOH, $-CHNNHCOR_9$, $-CHR_{10}N$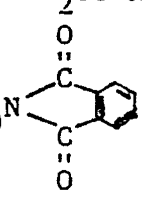 and $-COPO_3$,

wherein $R_1$ is a hydrogen atom or lower alkyl; $R_2$ is alkyl of 1 to 20 carbon atoms; $R_3$ is lower alkyl; $R_4$ is a hydrogen atom or lower alkyl; $R_5$ is lower alkyl; $R_6$ is lower alkyl; $R_7$ is lower alkyl; $R_8$ is lower alkyl; $R_9$ is lower alkyl; and $R_{10}$ is a hydrogen atom or lower alkyl.

The method of the present invention will become more apparent from the following more detailed description and the

accompanying drawings wherein:

Figure 1 is a perspective view of a smoking article, i.e. a filter cigarette, in accordance with the present invention;

Figure 2 is a cross-sectional view of a filter cigarette in accordance with one embodiment of the present invention;

Figure 3 is an enlarged view showing in cross section the filtering element of the filter cigarette of Figure 2;

Figure 4 is a cross-sectional view of a filter cigarette illustrating a further embodiment of the present invention;

Figure 5 is an enlarged view showing in cross section the filtering element of the filter cigarette of Figure 4;

Figure 6 is a cross-sectional view of a filter cigarette showing a further embodiment of the present invention;

Figure 7 is a further cross-sectional view showing a cigarette illustrating a further embodiment of the present invention;

Figure 8 is a cross-sectional view of the cigarette of Figure 1 in accordance with the embodiment of the present invention;

Figure 9 is a cross-sectional view of the cigarette of Figure 1 in accordance with another embodiment of the present invention.

In all of the figures, like numerals represent like elements

Figure 1 represents a filter cigarette 1 embodying a filtering device in accordance with the present invention. While this

figure illustrates a filter cigarette, it is important to note that the present invention is not limited thereto but is suitably applicable to the filtering of the smoke of cigars, pipes and similar smoking articles.

In addition, while this figure illustrates a filter cigarette having a filtering element, in accordance with the present invention, attached thereto, it is to be understood that the present invention is suitably applicable to the incorporation of the filtering element into a holder or similar attachment for use in the filtering of smoke of a cigarette or similar smoking article.

The filter cigarette 1 is shown in cross section in Figure 2. This figure illustrates the now-familiar filter-tip cigarette including a burnable paper wrapping or casing 3, a filling or smoke-producing material, e.g. tobacco 5, and a filter tip at the mouth end comprising a filter casing or tipping paper 7 and the filtering material 9. The filtering material 9 can comprise any of the standard materials now employed in the filtering of cigarettes and similar smoking articles. Thus the filtering material can comprise wadded cotton or rolled gauze, rolled, crimped cellulose sheet material, a matrix of charcoal or glass fibers, or a synthetic resinous material capable of entrapping the deleterious solid particles in the tobacco smoke. Dispersed within the filtering material 9 are tiny droplets 11 of vitamin A analog which material is uniformly dispersed throughout the filtering

medium 9. In this manner, when the smoke produced by the burning of the tobacco 5 within the burnable paper wrapping or casing 3 passes through the filter medium 9, such smoke will pick up the tiny droplets or an aerosol of vitamin A analog and carry them out of the filter of the cigarette to the smoker. The vitamin A analog will therefore pass with the smoke in the form of tiny droplets or an aerosol into the respiratory tract of the smoker.

Thus, there has been provided a new and unobvious method and/or filter cigarette for introducing vitamin A analog into the mouth and respiratory tract of a smoker with a view of providing another mode of introducing this valuable material into the respiratory tract of an individual.

In Figure 3, the filtering element of the present invention is shown in enlarged cross section. The droplets or an aerosol of vitamin A analog 11, dispersed within the filter medium 9 are of such a size that they are easily entrained by the smoke of the burning tobacco passing through the filtering element. While such a filtering element is shown in Figure 2 as forming the mouth portion of a conventional filter cigarette, it should be noted that the filtering element may be advantageously employed as a separate filtering element in a holder for a cigarette, cigar or similar smoking article. In addition, the filtering element can be advantageously employed as a separate filtering element located

in the body portion of a pipe. In all instances the smoke produced from the burning tobacco passing through the filtering element entrains the droplets of vitamin A analog 11, dispersed within the filter medium 9, such that the vitamin A analog is carried to the smoker along with the smoke.

An additional embodiment of the present invention is illustrated in Figure 4 which again shows a filtering device attached as the mouth portion of a conventional filter cigarette. The vitamin A analog within the filter medium 9 of the filtering element is not dispersed as tiny droplets within the filter medium 9 but is encapsulated in liquid or vapor form within a rupturable capsule 13 located within the filter medium 9. The rupturable capsule 13 containing the vitamin A analog is located within the filtering element in such a manner that the capsule can be easily ruptured upon the application of slight pressure. Such pressure can be easily supplied by the smoker prior to smoking the cigarette. The rupturable capsule 13 can be any organic material such as sugar, egg white, or a thin-walled plastic material which can be easily ruptured by the application of slight pressure. Exemplary materials include, for example, gelatin, polyethylene plastic, natural or synthetic rubber, or any other suitable material capable of rupturing by the application of slight pressure. The vitamin A analog 15 is maintained within the rupturable capsule 13 either in the liquid or vapor form, the liquid form being preferred. The vitamin A analog may also occur as the pure material or may be

employed as an aqueous solution located within the rupturable capsule 13. The term solution as used herein means solutions which are clear, show no dispersion visible to the naked eye, and remain so indefinitely. When an aqueous solution of the vitamin A analog is employed, the water which is released upon rupturing the capsule 13 additionally aids in moisturizing the smoke which is produced from the burning tobacco. In addition to vitamin A analog, it is important to note that various other additional ingredients can be incorporated within the rupturable capsule to add further desireable properties to the smoke produced. Thus, for example, additional health-benefitting vitamins as well as flavorants such as menthol or chlorophyll can be advantageously maintained within the rupturable capsule 13 along with the vitamin A analog of the present invention.

Figure 5 shows the filtering element of Figure 4 in enlarged cross section. Here again, while the filtering element is shown in Figure 4 as the mouth portion of a conventional cigarette, it should be known that such filtering element can be advantageously employed also as an independent filtering element located within the body of a pipe or similar tobacco-smoking article.

In addition, while the rupturable capsule or container 13 has been shown as a single large rupturable container or capsule within the filtering medium of Figures 4 and 5, the vitamin A analog and other materials can be conveniently encapsulated within a number of smaller rupturable capsules or containers located within

the filtering medium. In addition, such a rupturable container or capsule containing the vitamin A analog can be conveniently located within the tobacco portion of a cigarette or cigar should it be desired to obtain the benefits of the entrainment of the vitamin A within the smoke produced from the burning tobacco without the aid of a filtering medium. When the rupturable container or capsule 13 shown in Figures 4 and 5 is ruptured by slight pressure exerted by the fingers of the smoker, the liquid or vaporous vitamin A analog is released and dispersed throughout the filtering medium 9. In this way, the smoke produced from the burning of the tobacco of the cigarette or other tobacco-smoking article entrains the dispersed vitamin A analog as the smoke passes through the filtering element. In this manner the vitamin A analog is taken along with the smoke into the lungs and other organs of the smoker.

An alternative embodiment of the present invention is illustrated in Figure 6. In this embodiment, small capsules 17 of vitamin A analog are located on the inner face of the filter casing or tipping paper 7. The wall material of the capsules 17 can be prepared from the same material as set forth in Figures 4 and 5 for the rupturable container or capsule 13. These tiny capsules 17 containing vitamin A analog again are rupturable and the vitamin A analog is released upon slight pressure exerted by the smoker. When the vitamin A analog located within the small capsules 17 is released, it disperses throughout the filtering

medium 11 in such a manner that it is easily entrained by the smoke from the burning tobacco which passes through the filtering element

A still further embodiment of the present invention is shown in Figure 7. Here a smoking article such as a cigarette or cigar contains a fine dispersion 19 of vitamin A analog throughout the tobacco medium 5 of the cigarette or cigar. In this manner, vitamin A analog can be entrained in the smoke produced from the burning tobacco in the same manner as it could be entrained when employed as a dispersion within the filtering medium of a filter cigarette. This embodiment therefore allows for the obtaining of the beneficial results associated with the intake of vitamin A analog without the necessity of the employment of a filter to be used with a cigarette, cigar or other similar smoking article. While the present invention has been described primarily with respect to the incorporation of vitamin A analog as a dispersion or encapsulated, within the tobacco medium itself of such a tobacco-smoking article, it is noted at this time that a treatment of growing tobacco or cut tobacco prior to the manufacturing of the cigarettes or other smoking article with vitamin A analog will also add beneficial effects to the cigarettes or other smoking articles thus produced.

The cigarette 21 is shown in cross section in Figure 8. This figure illustrates a cigarette including a tobacco charge 25 within a burnable wrapper or casing 23.

Situated within the tobacco charge 25 is a rupturable capsule 27 containing the vitamin A analog. The vitamin A analog is maintained within the rupturable capsule 27 either in the form of a liquid or a vapor, the liquid form being preferred. The vitamin A analog may occur as the pure material or may be conveniently employed as an aqueous solution located within the rupturable capsule 27. The term "solution" as used herein means solutions which are clear, show no disperson visible to the naked eye, and remain so indefinitely.

When an aqueous solution of vitamin A analog is employed, the water which is released upon the rupturing of the capsule 27 by the influence of the hot smoke or by the heat of the burning tobacco charge aids in addition in moisturizing the smoke which is produced from the burning tobacco charge 25. The moisturizing effect of the release of the water by rupturing of the capsule 27 not only tends to reduce the temperature of the smoke passing into the mouth and respiratory tract of the smoker, but tends to aid the cilia, which are fine, hair-like projections or cells on the linings of the bronchial tubes.

It is noted that in addition to the presence of an aqueous solution of vitamin A analog within the rupturable capsule 27, it is also possible to include other health-benefitting agents,

i.e., other vitamins, specifically vitamin C and vitamin E, which have been found to act favorably in conjunction with vitamin A. In addition, various flavoring agents such as menthol, pectin or chlorophyll can be added in order to produce a more pleasing flavor in the tobacco smoke.

The rupturable capsule 27 is composed of any natural or synthetic material which is capable of being ruptured by melting or disintegration or other decomposition at the temperature of the tobacco smoke or burning tobacco charge. In this respect, suitable materials include, for example, gelatin, solidified egg white, thermoplastic synthetic resin which melt at a sufficiently low temperature, and thermosetting synthetic resins which disintegrate at a temperature below the temperature of the tobacco smoke, or heat of the tobacco charge.

The tobacco smoke passing through the tobacco charge or the heat of the burning tobacco charge will cause the rupturable capsule 27 to rupture by melting or disintegrating or other decomposition, thereby releasing the vitamin A analog. The smoke passing through the tobacco will therefore entrain or disperse the vitamin A analog and other materials released from the rupturable capsule 27 in the form of tiny droplets or an aerosol and carry such entrained or dispersed material into the mouth and respiratory tract of the smoker. The smoking article, therefore, allows for a new and improved method or mode for the introduction of the valuable vitamin A analog into the respiratory tract of the smoker where it can have its beneficial effects.

Figure 9 illustrates a further embodiment of the present invention. This figure again illustrates a conventional cigarette wherein a tobacco charge 25 is present within a burnable wrapper or casing 23. Located within the tobacco charge 25 is a multiplicity of small capsules, e.g. microcapsules 29 containing vitamin A analog.

The microcapsules 29 within the filtering material 25 are composed of the same material set forth for the capsule 27 in Figure 8. Thus the microcapsules are composed of a material which is capable of being ruptured by melting, disintegration or other decomposition either under the influence of hot tobacco smoke or by the heat of the burning tobacco charge.

The term "microcapsule" as employed in reference to Figure 9 relates to capsules which are small and numerous enough so that the tobacco charge contains a multiplicity thereof which, when ruptured, release a pre-determined amount of vitamin A analog to be dispersed or entrained within the smoke emanating from the smoking article so as to be passed with said smoke into the respiratory tract of the smoker.

While the embodiment shown in Figures 8 through 9 have been shown as separate embodiments of the present invention, it is, of course, obvious that these embodiments can be successfully combined in the production of advantageous smoking articles.

It is pointed out with respect to the figures herein described that any number of rupturable capsules can be employed as

long as one such capsules is present within the smoking article. Thus, while Figure 8 shows the employment of merely one large capsule containing vitamin A analog and Figure 9 illustrates the employment of a multiplicity of small capsules within the tobacco charge, it is obvious that any number of capsules can be utilized. Thus, for example, it is possible to employ two, three, four, etc., capsules ranging in size from very large capsules as pictured in Figure 8 to small or microcapsules as shown in Figure 9.

In addition, it is possible that under some circumstances a stabilizing agent may be added to the capsules containing vitamin A analog so as to prevent the same from degrading. In this respect any of the conventional oxidation stabilizers can be successfully employed in the vitamin A analog composition within the rupturable capsules.

The vitamin A analogs as used in the method of the subject invention are known in the art and have been the subject of extensive studies regarding their potential effectiveness and especially, a prophylactic effect and in some instances, a therapeutic effect on tumors, including not only those associated with lung cancer, but also such maladies as sarcomas, adenocarcinomas, and prostate, esophagus, bladder, colon and skin cancers. The vitamin A analogs are referred to in the art as retinoids and are chemically and/or related to vitamin A, but do not possess the toxic side effects associated with large dosages of vitamin A. Vitamin A, also called all-trans-β-vitamin A alcohol or all-trans-β-retinol is illustrated below, including the numbering system of

the carbon atoms:

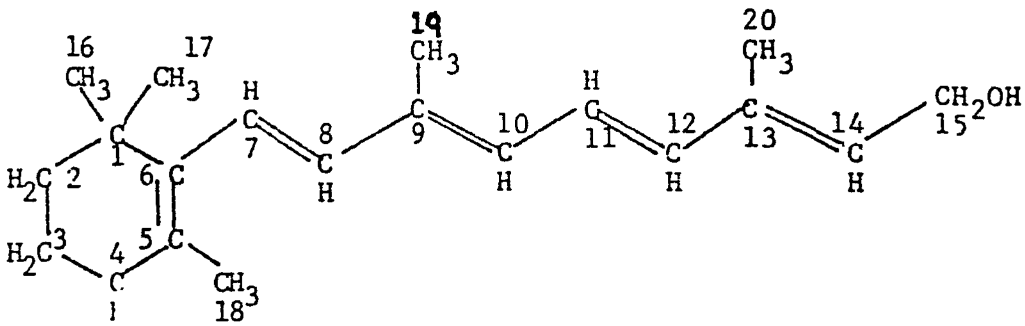

The vitamin A formula is more conveniently drawn as follows:

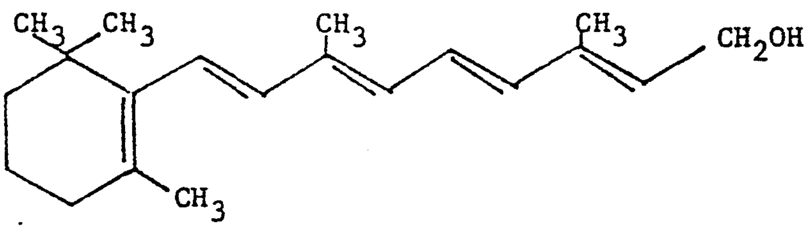

Basically, the parent vitamin A molecule can be considered to consist of three sub-sections:

a. hydrocarbon ring 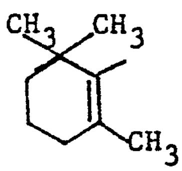 (β-cyclohexenyl)

b. hydrocarbon side chain 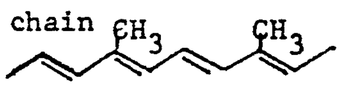 (all-trans form)

c. terminal group 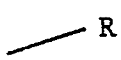

In the analogs within the scope of the subject invention, the hydrocarbon ring is preferably the β-cyclohexenyl group (1,1, 5 trimethyl β-cyclohexenyl) moiety, although the following

hydrocarbon ring moieties can also be used in preparing the vitamin A analog of the subject invention:

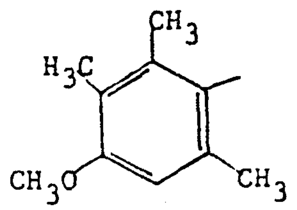

trimethylmethoxyphenyl analog

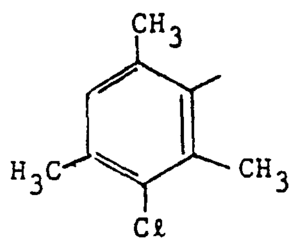

chlorotrimethylphenyl analog

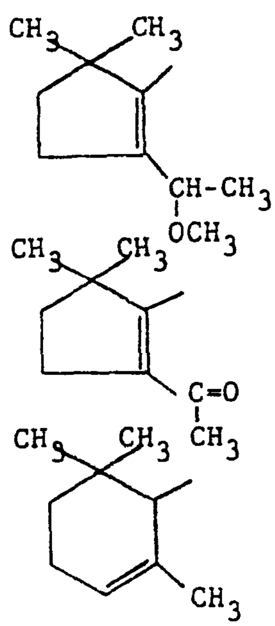

1-methoxyethyl cyclopentenyl analog

methylketo cyclopentenyl analog (dimethylacetyl cyclopentenyl analog

the γ-cyclohexenyl analog

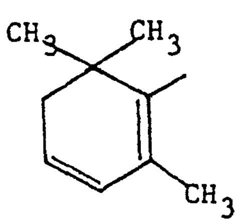

the cyclohexenyl moiety found in vitamin $A_2$ (additional unsaturated double bond at 3-4 position)

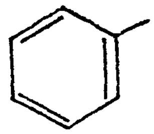

phenyl analog

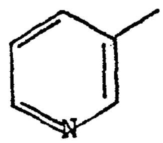

pyridyl analog

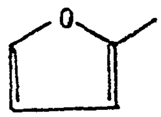

furyl analog

Similarly, while the vitamin A analogs of the subject invention include the all-trans form as shown above, the following modifications of the hydrocarbon side chain illustrated with respect to the above formulas (I), (II) or (III) can be satisfactorily used in the subject invention. Specific examples of suitable hydrocarbon side chains which can be used in the subject invention include the following:

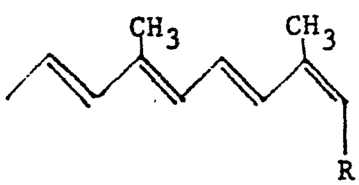

13-cis analog

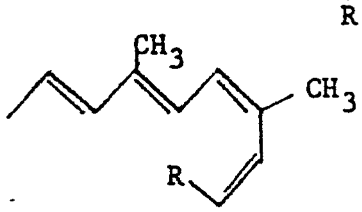

11-cis analog

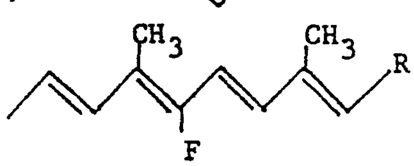

10-fluoro analog

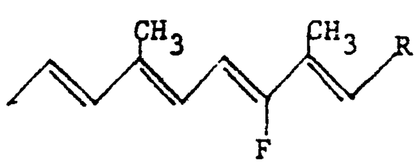

12-fluoro analog

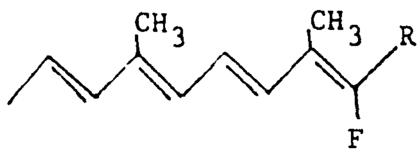

14-fluoro analog

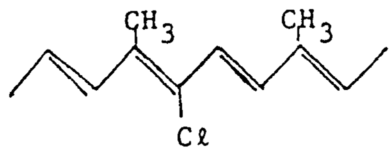

10-chloro analog

In fact, the 11-cis and 13-cis analogs are believed to be especially preferable with respect to their low degree or substantial absence of toxicity and their effectiveness at low dosage rates.

The terminal group R of the vitamin A analog can be any one of the following groups:

| | |
|---|---|
| -CHO | hydrogen atom or lower alkyl |
| $-COOR_1$ | alkyl of 1 to 20 carbon atoms |
| $-R_2-OC(=O)-R_3$ | lower alkyl |
| $-CONHR_4$ | hydrogen atom or lower alkyl |
| $-R_5OR_6$ | lower alkyl |
| $-R_7NHCOR_8$ | lower alkyl |
| -CHNOH | lower alkyl |
| $-CHNNHCOR_9$ | lower alkyl |
| 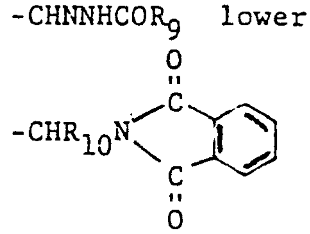 | lower alkyl |
| $-COPO_3$ | hydrogen atom or lower alkyl |

Examples of preferred terminal groups include the following:

| | |
|---|---|
| $CHO$ | aldehyde analog |
| $COOH$ | acid analog |
| $CH_2OCOCH_3$ | acetate analog |
| $(CH_2)_{14}OCOCH_3$ | palmitate analog |
| $CONHCH_3$ | methyl amide analog |
| $CONHC_2H_5$ | ethyl amide analog |
| $CONH(CH_2CH_3)_2$ | diethyl amide analog |
| $CH_2OCH_3$ | methyl ether analog |
| $CH_2NHCOCH_3$ | N-acetyl amine analog |
| $CHNOH$ | oxime analog |
| $CHNNHCOCH_3$ | acetyl hydrazone analog |
| $CH_2OC_4H_9$ | butyl ether analog |
| $COOCH_3$ | methyl ester of acid analog |
| $COOC_2H_5$ | ethyl ester of acid analog |
| 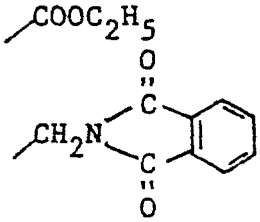 | N-phthalimide analog |
| $COPO_3$ | phosphate analog |

Examples of specific vitamin A analogs within the scope of the invention include the following compounds:

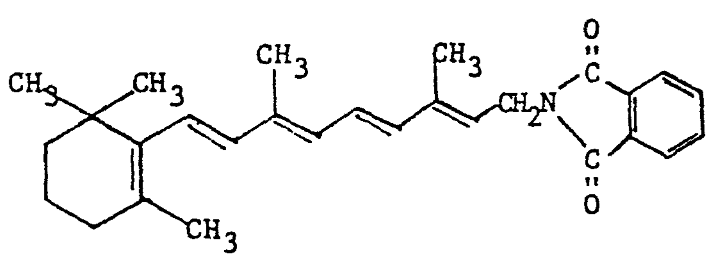

N-Retinyl Phthalimide

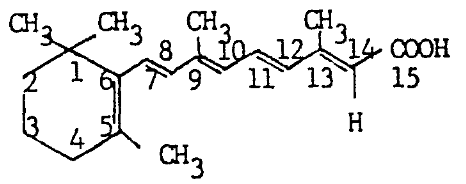

β-retinoic acid

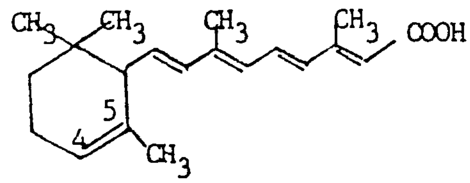

α-retinoic acid

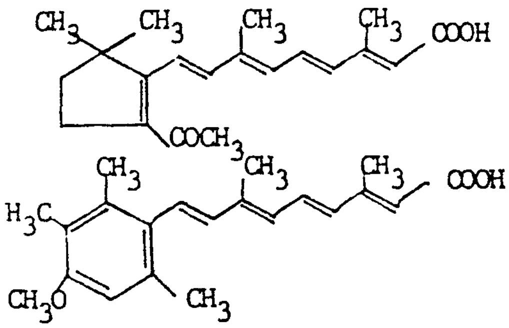

cyclopentenyl analog of retinoic acid

trimethylmethoxyphenyl analog of retinoic acid

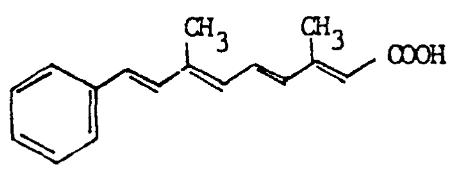

phenyl analog of retinoic acid

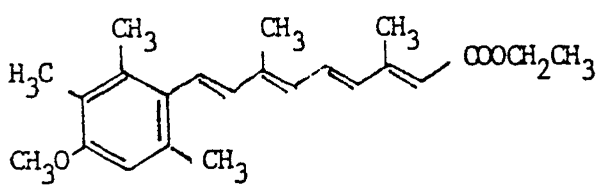

trimethylmethoxyphenyl analog of ethyl retinoate

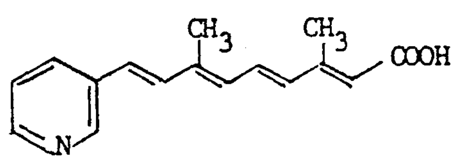

pyridyl analog of retinoic acid

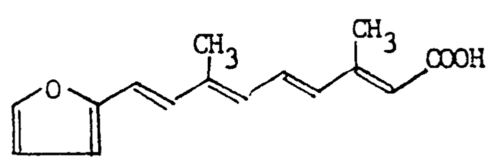

furyl analog of retinoic acid

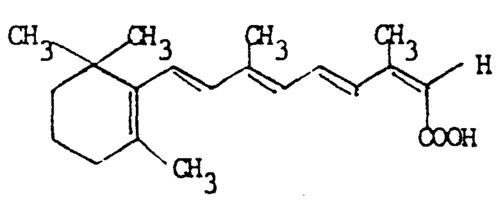

13-cis-retinoic acid

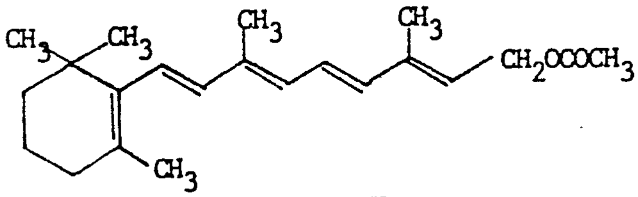

β-retinyl acetate

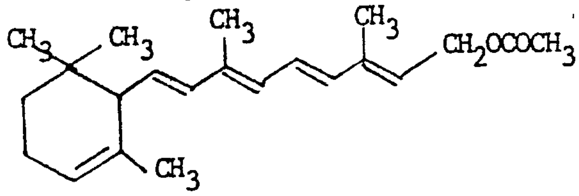

α-retinyl acetate

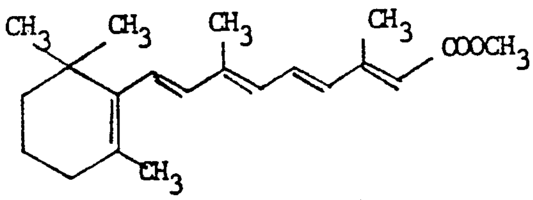

methyl retinoate

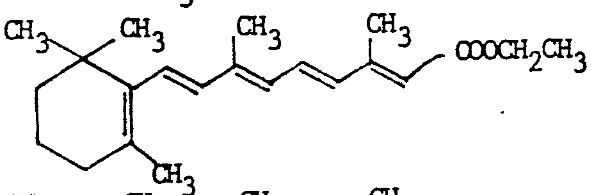

ethyl retinoate

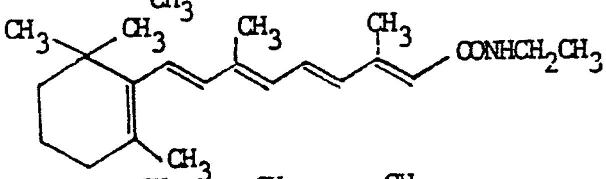

N-ethylretinamide

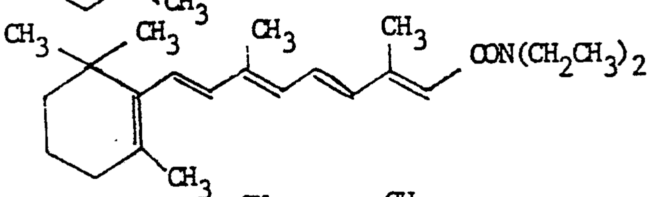

N,N-diethylretinamide

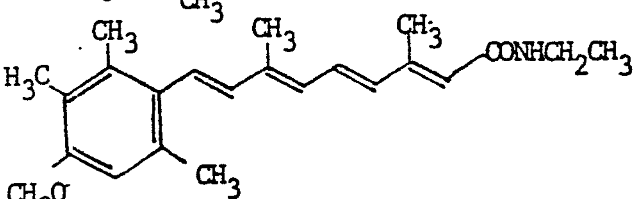

trimethylmethoxyphenyl
analog of N-ethylretinamide

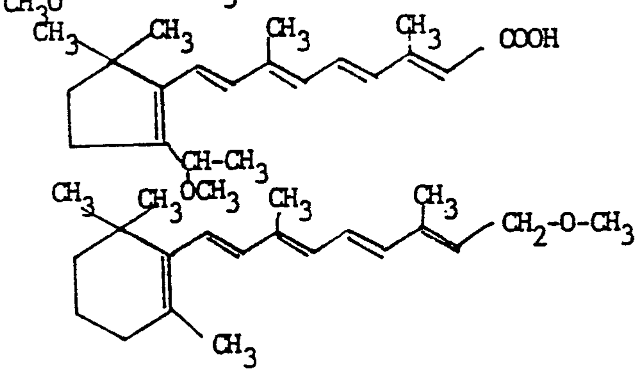

1-methoxyethyl cyclopentenyl
analogue of retinoic acid

retinyl methyl ether

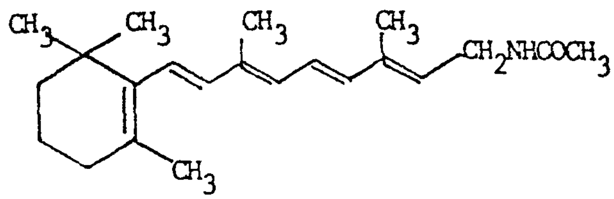

N-acetylretinylamine

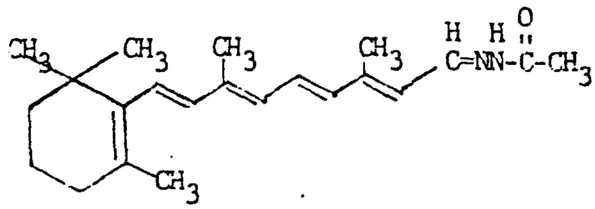

retinal acetyl hydrazone

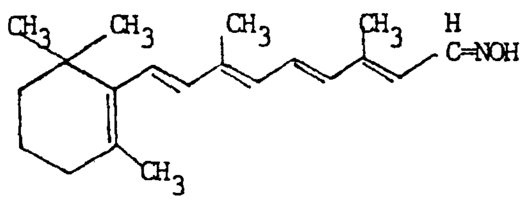

retinal oxime

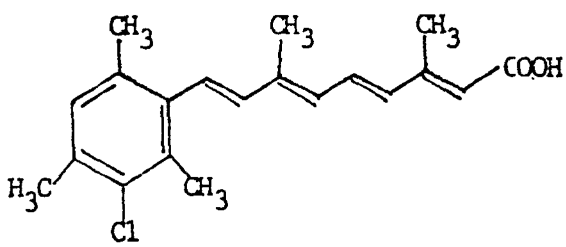

chlorotrimethylphenyl analogue of retinoic acid

It is therefore seen that the method of the subject invention provides a technique for administering safe and effective dosages of analogs of vitamin A in much lower, non-toxic quantities than needed via the oral route to obtain the same or equivalent prophylactic or therapeutic effects. The method of this invention is particularly advantageous for the cigarette smoker. The method of delivery according to the subject invention would not evoke any negative psychological effect created by the need to take a separate medication. Secondly, the cigarette smoker need not be an active

participant in the process, since as long as he smoked cigarettes containing the vitamin A analogs according to the subject invention, he would receive the benefit of the retinoid substance without any need to remind himself to take a pill. Thirdly, the dosage received would be in direct proportion to the number of cigarettes smoked. The heavier smoker, who probably would need more of the beneficial effects of the retinoid would receive more of the vitamin A analog than the person who smoked only a few cigarettes a day. Thus, the dosage would be directly related to the need and would not be arbitrarily set at any maximum or minimum levels, which need not be appropriate to all individuals.

Also, while the foregoing description of the process of the subject invention was given principally in terms of cigarettes or similar smoking articles associated with tobacco or the like, it should be recognized that the method of the subject invention can be used in conjunction with cigarettes or other smoking article based on tobacco substitutes, whether naturally occurring substances or artificially produced substances.

0003064

What is claimed is:

Claim 1. A method of introducing a natural or synthetic analog of vitamin A into the mouth and respiratory tract of a smoker which comprises

locating at least one heat or pressure rupturable capsule containing a controlled amount of a vitamin A analog selected from the group consisting of compounds of the following formulas:

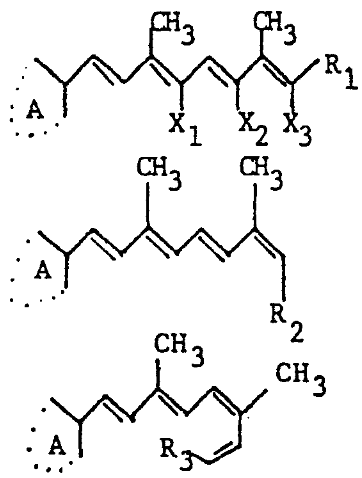

(I)

(II)

and

(III)

wherein in the ring A, 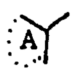, is a five or six membered, mono- or di-unsaturated or aromatic hydrocarbon ring, which may include a nitrogen or oxygen hetero atom in the ring, and which may include one or more substituents selected from the group consisting of lower alkyl, lower alkoxy, halogen, lower alkoxyalkyl and lower acyl;

$X_1$, $X_2$ and $X_3$ are each a hydrogen atom or one of them may be a halogen atom; and

R is a member selected from the group consisting of -CHO, $-COOR_1$, $-R_2-O\overset{O}{\overset{\|}{C}}-R_3$, $-CONHR_4$, $-R_5OR_6$, $-R_7NHCOR_8$, -CHNOH, $-CHNNHCOR_9$,

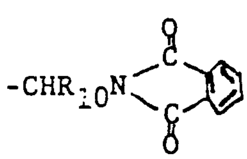 and $-COPO_3$,

wherein $R_1$ is a hydrogen atom or lower alkyl; $R_2$ is alkyl of 1 to 20 carbon atoms; $R_3$ is lower alkyl; $R_4$ is a hydrogen atom or lower alkyl; $R_5$ is lower alkyl; $R_6$ is lower alkyl; $R_7$ is lower alkyl; $R_8$ is lower alkyl; $R_9$ is lower alkyl; and $R_{10}$ is a hydrogen atom or lower alkyl; in at least the mouth end of a cigarette having a tobacco charge;

rupturing each vitamin A analog containing capsule immediately before or during smoking to thereby release said vitamin A analog in the form of tiny droplets or an aerosol for exposure to the smoke passing through said cigarette; and smoking said cigarette whereby said tiny droplets or aerosol of said vitamin A analog will be carried in the smoke to the mouth and respiratory tract of the smoker.

Claim 2. The method of claim 1 wherein a single pressure rupturable capsule is used, and wherein said capsule is ruptured by application of pressure thereto immediately before smoking.

Claim 3. The method of claim 2 wherein said cigarette further comprises a filter medium downstream from said tobacco charge, and said capsule is in contact with said filter medium,

Claim 4. The method according to claim 1 wherein a plurality of pressure rupturable capsules are used, and wherein at least the majority of said capsules are ruptured by application of pressure thereto immediately before smoking.

Claim 5. The method according to claim 4 wherein said cigarette further comprises a filter medium downstream from said tobacco charge, and at least the majority of said capsules are in

contact with and uniformly distributed throughout said filter medium.

Claim 6. The method according to claim 1 wherein a plurality of heat rupturable capsules are used, said capsules being relatively uniformly distributed over the length of said cigarette, said capsules being rupturable under the influence of either hot tobacco smoke or the heat of the burning tobacco charge.

Claim 7. The method of claim 6 wherein said heat rupturable capsules comprise a multiplicity of microcapsules.

Claim 8. The method of claim 1 wherein in said vitamin A analog said ring A is selected from the group consisting of

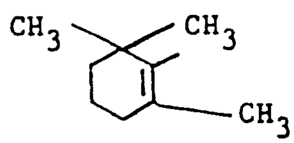 ,

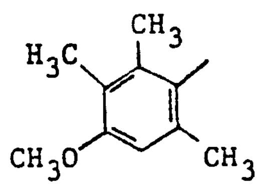 , 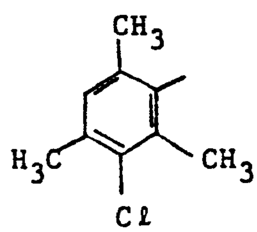 ,

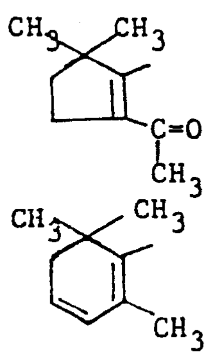 , 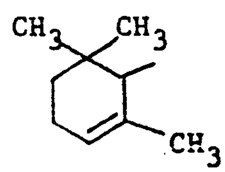 ,

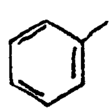 , 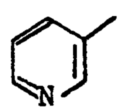 and

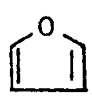 .

Claim 9. The method of claim 1 wherein in said vitamin A analog R is selected from the group consisting of -CHO, -COOH, $-CH_2OCOCH_3$, $-(CH_2)_{14}OCOCH_3$, $-CONHCH_3$, $-CONHC_2H_5$, $-CONH(CH_2CH_3)_2$, $-CH_2OCH_3$, $-CH_2NHCOCH_3$, -CHNOH, $-CHNNHCOCH_3$, $-CH_2OC_4H_9$, $-COOCH_3$, $-COOC_2H_5$, 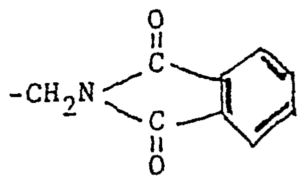 and $-COPO_3$.

Claim 10. The method of claim 1 wherein said vitamin A analog is selected from the group consisting of N-retinyl phthalimide, β-retinoic acid, α-retinoic acid, cyclopentenyl analog of retinoic acid, trimethylmethoxyphenyl analog of retinoic acid, phenyl analog of retinoic acid, trimethylmethoxyphenyl analog of ethyl retinoate, pyridyl analog of retinoic acid, furyl analog of retinoic acid, 13-cis-retinoic acid, β-retinyl acetate, α-retinyl acetate, methyl retinoate, ethyl retinoate, N-ethylretinamide, N,N-diethylretinamide, trimethylmethoxyphenyl analog of N-ethylretinamide, 1-methoxyethyl cyclopentenyl analogue of retinoic acid, retinyl methyl ether, N-acetylretinylamine, retinal acetyl hydrazone, retinal oxime, chlorotrimethylphenyl analogue of retinoic acid.--

0003064

1/1

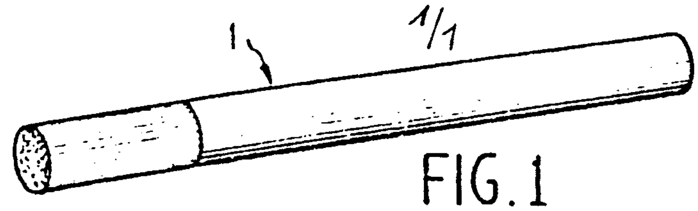


FIG.1

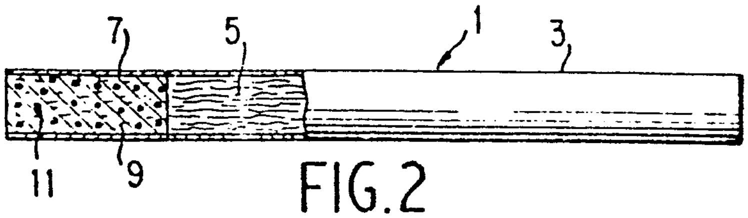


FIG.2

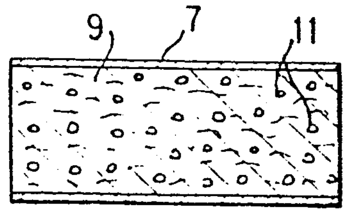


FIG.3

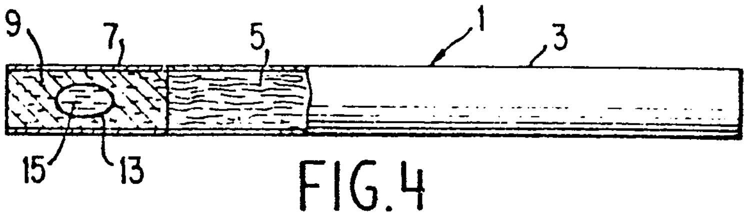


FIG.4

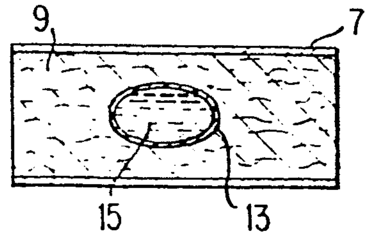


FIG.5

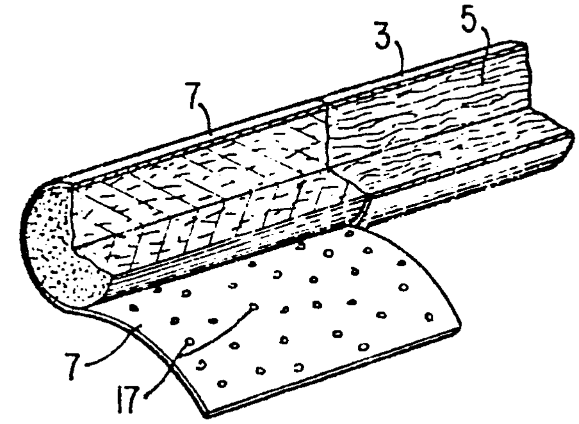


FIG.6

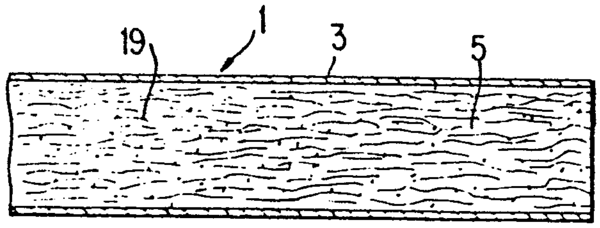


FIG.7

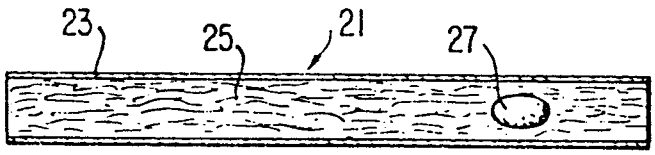


FIG.8

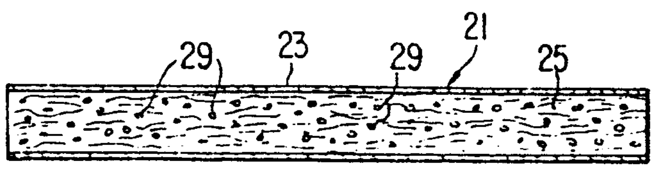


FIG.9